# EUROPEAN PATENT APPLICATION

(11) **EP 4 566 597 A1**
(43) Date of publication of application: **11.06.2025**
(21) Application number: 23383259.1
(22) Date of filing: 05.12.2023
(51) Int. Cl.: A61K 31/22, A61K 31/366, A61K 31/40, A61K 31/404, A61K 31/47, A61K 31/505, A61K 45/06, A61P 7/02

(54) **STATINS USEFUL IN THE TREATMENT OF SPLACHNIC VASCULAR DYSFUNCTIONS**

(71) Applicant: Hospital Clínic de Barcelona, 08036 Barcelona (ES); Fundació de Recerca Clínic Barcelona-Institut d'Investigacions Biomèdiques August Pi I Sunyer, 08036 Barcelona (ES)
(72) Inventor: HERNÁNDEZ GEA, Virginia, 08950 ESPLUGUES DE LLOBREGAT (ES); SHALABY, Sarah, 08012 BARCELONA (ES); ANTON MARTÍNEZ, Aina, 08026 BARCELONA (ES); CAMPRECIÓS FIGUERAS, Genís, 08960 SANT JUST DESVERN (ES); GARCIA PAGÁN, Joan Carles, 08980 SANT FELIU DE LLOBREGAT (ES)
(74) Representative: ZBM Patents - Zea, Barlocci & Markvardsen

(57) **Abstract**

It relates to statins or their pharmaceutically acceptable salts for use in the prevention and/or treatment of splanchnic vascular dysfunctions in a patient which is a mammal wherein the prevention and/or treatment comprises inhibiting and/or reverting endothelial-to-mesenchymal transition of endothelial cells.

## Description

The present invention relates to statins useful in the prevention and/or treatment of splachnic vascular dysfunctions.

### BACKGROUND ART

The liver has a unique circulatory system evolved to protect against ischemic injury; 75% of blood inflow comes through the portal vein and the remaining 25% through the hepatic artery. The portal vein, which is the main blood vessel in the splachnic venous system (SVS), carries blood from organs in the abdomen (stomach, pancreas, spleen, gallbladder, small and large intestine) to the liver, where it is filtered and processed before it returns to the body's general circulation. Smaller veins in the SVS collect blood from all the organs in the abdomen and deliver it to the portal vein. The SVS has several particularities when compared to the systemic venous system. In healthy conditions, SVS represents a unique vascular environment due to its low-pressure, slow flow and high-volume hemodynamics and the PV compliance is very high (the vessel can be easily distended to compensate variations in portal vein flow) making thrombosis a rare event.

Cirrhosis has been suggested to be a vascular liver disease, since it is associated with many changes at the circulatory level, for instance, decreased portal vein blood flow velocity has been suggested as a risk factor for Portal Vein Thrombosis (PVT) development. Due to changes in the pro- and anti-coagulant factors mainly produced in the liver, cirrhosis is associated with an instable homeostatic rebalance leading to a hypercoagulability state.

PVT is defined as the presence of a thrombus in the portal vein (PV) or its branches, which may extend to the splanchnic territory. As mentioned above, PVT is a rare event in the general population (global prevalence of 1%), whereas in patients with cirrhosis (CH), up to 26% of patients may suffer from this condition and it has a prevalence of more than 17% among patients awaiting liver transplantation, according to Nicoar 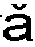 -Farc 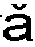 u, O., *et al.,* in "New Insights into the Pathogenesis, Risk Factors, and Treatment of Portal Vein Thrombosis in Patients with Cirrhosis", *Seminars in Thrombosis* & *Hemostasis,* 2020, Vol. 46, p. 673-681. PVT is hence generally associated with impaired liver function and worsening of portal hypertension (PH), the final driver of liver decompensations and liver-related mortality. PH is a common complication of cirrhosis and its treatment has hence received increasing attention in clinical research. Some studies, as disclosed by Wan S., et al., in "Systematic review with a meta-analysis: clinical effects of statins on the reduction of portal hypertension and variceal haemorrhage in cirrhotic patients", BMJ Open, 2019, Vol. 9, Issue 7, have shown that statins are safe for cirrhotic patients and may decrease portal hypertension alone or in combination with non-selective betablockers (NSBB). However, these results still lack confirmation based on further and larger randomized controlled trials (RCTs).

Although the reasons are still largely unknown, impaired SVS rarely responds to anticoagulant (AC) treatment, meaning that a considerable amount of patients receive an ineffective treatment and are hence more susceptible of facing a poorer prognosis. Many patients suffering from liver disorders, such as cirrhosis, may be subject to a liver transplant (LT) as the only remaining treatment. By occluding the vessel, chronic PVT may increase portal hypertension (PHT) and its complications (variceal bleeding, ascites) and, when extensive, may impair the needed anastomosis for LT (see Bhangui, P., et al., in "Current management of portal vein thrombosis in liver transplantation", International Journal of Surgery, Vol. 82, 2020, p.122-127). PVT hence adds technical complexity to the LT procedure and increases the risk of developing PVT post-surgery, especially in the first days post-LT, with patients having increased peri and post-transplant morbidity and mortality. Thus, PVT remains a frequent and unresolved problem for patients with cirrhosis, with an incidence ranging from 10% to 26%, highlighting the need for an effective treatment to improve prognosis and quality of life of these patients.

WO2018221480A1 discloses a PVT therapeutic agent, namely antithrombin III, which is a glycoprotein produced by the liver and whose production may hence be impaired in patients suffering from liver pathologies. It may be hence useful in the treatment of PVT in that it inactivates proteases involved in coagulation, however, this implies that PVT has to be caused by coagulation dearagement. Therefore, this agent would not be useful in the cases having a different cause also leading to PVT.

Up to date, anticoagulation is the only medical treatment recommended for PVT (see for instance, De Franchis, R., et al., in "Baveno VII - Renewing consensus in portal hypertension", Journal of Hepatology, vol. 76, 2022, p. 959-974). However, unlike other veins, up to 60% of PVT patients fail to achieve recanalization (see Anton, A., et al., in "The Pathophysiology of Portal Vein Thrombosis in Cirrhosis: Getting Deeper into Virchow's Triad", Journal of Clinical Medicine, 2022, vol. 11, Article number 800). Moreover, re-thrombosis occurs in up to one-third of patients when anticoagulation is withdrawn - a higher rate than other veins - and the risk of thrombosis persists despite improvements in liver function. The composition of the thrombus in the SVS remains quite enigmatic, and despite its venous nature, there is a suggested unique composition that underlies the distinctive pathogenesis of this condition (see Driever, E. G., et al., in "Nonmalignant portal vein thrombi in patients with cirrhosis consist of intimal fibrosis with or without a fibrin-rich thrombus", Hepatology, 2022, Vol. 75, p. 898-911).

On the other hand, it is widely known that venous thrombosis is governed by a triad of pathophysiological factors: hypercoagulability, changes in blood flow and endothelial dysfunction (the so-called Virchow's triad). However, in the case of patients with cirrhosis, the exact contribution of each of these factors to the development of PVT still remains unclear, leaving the exact pathophysiology of PVT largely unknown.

Anticoagulants, including antithrombin III , which are the only state of the art to treat PVT, fail to accomplish the desired effect in a great number of patients sufferering from PVT, specially in cirrhotic patients. To date, anticoagulants are the only approved pharmacological treatment for PVT and, since they are not effective in certain patients, these patients are exposed to their secondary effects, which may be lethal (such as hemorrhages), for no reason. Moreover, a reduction of PH does not necessarily help reduce PVT or its risks in patients suffering from PVT, in particular in cirrhotic patients, since PV wall thickening is one of the underlying problems (see Driever, E. G., et al., Hepatology, 2022, Vol. 75, p. 898-911).

Accordingly, there is still the need of providing a safer and more effective therapeutic option for patients suffering from PVT, in particular, for cirrhotic patients, given that this disorder can impair patient outcome if left untreated.

### SUMMARY OF THE INVENTION

The present inventors have found that the endothelial-to-mesenchymal transition (EndMT) of endothelial cells, in particular, portal vein endothelial cells, leads to anatomical changes in the splachnic venous system, which is one of the main causes of portal vein thrombosis.The present inventors have also found that statins or pharmaceutically acceptable salts thereof inhibit such endothelial-to-mesenchymal transition (EndMT) of endothelial cells, providing an effective therapeutic option of preventing and/or treating splanchnic vascular dysfunctions, in particular portal vein thrombosis.

Inventors have also found that PVECs during cirrhosis undergo endothelial-to-mesenchymal transition (EndMT), with maximal expression in patients with CH and PVT. This transition may cause, for instance, the thickening of portal vein wall, hence reducing the lumen and having an impact on blood flow and portal hypertension. As far as the inventors know, no EndMT inhibitory activity in PVECs has been suggested in the prior art for statins. Statins are widely used drugs that have already shown its safety and even efficacy in treating cirrhosis (e.g., ameliorating PH) but they have never been indicated before for preventing and/or treating PVT complication.

Accordingly, the present invention relates to statins or pharmaceutically acceptable salts thereof for use in the prevention and/or treatment of splanchnic vascular dysfuntions in a patient which is a mammal, including a human being, wherein the prevention and/or treatment comprises inhibiting and/or reverting endothelial-to-mesenchymal transition of endothelial cells.

### BRIEF DESCRIPTION OF DRAWINGS

FIG.1 shows representative images of H&E staining of portal veins of control patients (LT donors), cirrhotic patients without PVT and cirrhotic patients with PVT.
FIG.2 shows measurements of TI (tunica intima) and TM (tunica media) extrahepatic portal vein samples from control patients (LT donors), cirrhotic patients without PVT and cirrhotic patients with PVT.
FIG.3 shows representative images of portal vein wall composition of control patients (LT donors), cirrhotic patients without PVT and cirrhotic patients with PVT, using Movat Pentachrome Stain Kit.
FIG.4 shows collagen, fibrin, mucin, muscle and elastic fibers content in control patients (LT donors), cirrhotic patients without PVT (CH) and cirrhotic patients with PVT (PVT).
FIG.5 shows fGSEA results from the comparison between CT_PVECs and CH_PVECs.
FIG. 6 shows fGSEA results from the comparison between CH_PVECs and CH_PVT_PVECs.
FIG. 7 shows hallmark genes of EndMT expression, presented in transcripts per million reads (tpm) for the three groups of patients. "Cont" in this figure means continuation.
FIG.8 shows two graphs of the quantification of CD68 and Cd3 infiltration in the portal vein wall of control patients and cirrhotic patients with or without PVT.
FIG. 9 shows PCR results from the *in vitro* treatment of PVECs with Simvastatin.
FIG. 10 shows Western blot results from the *in vitro* treatment of PVECS with Simvastatin.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply throughout the description and claims.

The expression "therapeutically effective amount" as used herein, refers to the amount of a compound that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations.

The expression "pharmaceutically acceptable excipients or carriers" refers to pharmaceutically acceptable materials, compositions, or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

The term "patient" refers to a person who is designated to receive a medical treatment, therapy, or service. The term patient may be extended to an animal when used within the context of the animal receiving veterinary treatment or services.

The expression "pharmaceutically acceptable salt" refers to any salt formed, provided that it is a non-toxic salt, in the present case the salt is formed with an inorganic base selected from the group consisting of sodium hydroxide, potassium hydroxide, calcium hydroxide, magnesium hydroxide, ammonium hydroxide; or an or organic base selected from the group consisting of methylamine, diethylamine, triethylamine, triethanolamine, ethylenediamine, tris(hydroxymethyl)methylamine, guanidine, choline, cinchonine and the like; or an amino acid such as lysine, arginine, alanine, and the like.

As mentioned above, the present invention relates to statins or pharmaceutically acceptable salts thereof for use in the prevention and/or treatment of splanchnic vascular dysfunctions in a patient which is a mammal, including a human being, where the prevention and/or treatment comprises inhibiting and/or reverting endothelial-to-mesenchymal transition of endothelial cells.

This aspect may also be formulated as the use of a statin or a pharmaceutically acceptable salt thereof, as defined above, for the preparation of a medicament for the prevention and/or treatment of splanchnic vascular dysfunctions in a patient which is a mammal, including a human being, where the prevention and/or treatment comprises inhibiting and/or reverting endothelial-to-mesenchymal transition of endothelial cells.

The present invention also relates to a method for the prevention and/or treatment of splanchnic vascular dysfunctions, comprising administering a therapeutically effective amount of a statin or a pharmaceutically salt thereof, as defined above, together with pharmaceutically acceptable excipients or carriers, in a patient which is a mammal, including a human being.

Splachnic venous system dysfunctions may be caused by related organ pathologies. For instance, as it is illustrated in the Examples, during liver damage, such as cirrhosis, tunica intima of the portal vein suffers from fibrosis and thickening, derived from endothelial-to-mesenchymal transition of portal vein endothelial cells. This has an impact in portal vein blood flow and may promote the development of PVT in these patients. For the first time, as far as the inventors know, PVECs (portal vein endothelial cells) from patients with cirrhosis (CH) with and without PVT were isolated and compared. As already explained, the present inventors found that one of the most significantly altered processes in PVT was the up-regulation of genes related to endothelial to mesenchymal transition (EndMT) pathway. The examples also illustrate that when PVECs of cirrhotic patients were treated *in vitro* with simvastatin, an increase in endothelial markers was observed, together with a decrease in mesenchymal markers, signalling statins' active role in the inhibition of EndMT.

Thus, in a particular embodiment, the statins or the pharmaceutically acceptable salts for use as defined above, are those where the splanchnic vascular dysfuntion is portal vein thrombosis and the endothelial cells are portal vein endothelial cells.

The consequences of EndMT in endothelial cells are hence anatomical changes in the different veins of the splachnic venous system, creating a favourable enviroment for the development of splachnic venous dysfunctions, such as portal vein thrombosis. Thus, in a particular embodiment, the statins or the pharmaceutically acceptable salts for use as defined above, are those where the inhibition and/or revertion of the endothelial-to-mesenchymal transition of endothelial cells inhibits and/or reverts anatomical changes in the splachnic venous system. In a more particular embodiment, the anatomical change is portal vein wall thickening. Such thickening appear as a result of tunica intima thickening due to EC proliferation and important ECM deposition changes. Also, PV wall remodelling showed important fibrosis and tunica media degeneration. Moreover, these histological changes promoting, in particular, portal vein wall thickening, were even more exacerbated in patients already suffering from PVT. Thus, in a more particular embodiment, the anatomical change is portal vein wall thickening.

By halting PV wall thickening, hence resulting in a wider lumen of the PV, reducing shear stress in the splachnic territory. Thus, in a particular embodiment the statins or the pharmaceutically acceptable salts thereof for use as defined above, are those where the prevention and/or treatment further comprises reducing portal vein hypertension.

Statins are structurally similar compounds exhibiting similar properties, thus, in a particular embodiment, the statins or the pharmaceutically acceptable salts thereof, for use as described above, are selected from the group consisting of:
Simvastatin, with IUPAC name [(1S,3R,7S,8S,8aR)-8-[2-[(2R,4R)-4-hydroxy-6-oxooxan-2-yl]ethyl]-3,7-dimethyl-1,2,3,7,8,8a-hexahydronaphthalen-1-yl] 2,2-dimethylbutanoate and CAS number 79902-63-9;
Atorvastatin, with IUPAC name (3R,5R)-7-[2-(4-fluorophenyl)-3-phenyl-4-(phenylcarbamoyl)-5-propan-2-ylpyrrol-1-yl]-3,5-dihydroxyheptanoic acid and CAS number 134523-00-5;
Fluvastatin, with IUPAC name (*E*,3*R*,5*S*)-7-[3-(4-fluorophenyl)-1-propan-2-ylindol-2-yl]-3,5-dihydroxyhept-6-enoic acid and CAS number 93957-54-1;
Lovastatin, with IUPAC name [(1S,3R,7S,8S,8aR)-8-[2-[(2R,4R)-4-hydroxy-6-oxooxan-2-yl]ethyl]-3,7-dimethyl-1,2,3,7,8,8a-hexahydronaphthalen-1-yl] (2S)-2-methylbutanoate and CAS number 75330-75-5;
Pitavastatin, with IUPAC name (E,3R,5S)-7-[2-cyclopropyl-4-(4-fluorophenyl)quinolin-3-yl]-3,5-dihydroxyhept-6-enoic acid and CAS number 147511-69-1;
Pravastatin, with IUPAC name (3R,5R)-7-[(1S,2S,6S,8S,8aR)-6-hydroxy-2-methyl-8-[(2S)-2-methylbutanoyl]oxy-1,2,6,7,8,8a-hexahydronaphthalen-1-yl]-3,5-dihydroxyheptanoic acid and CAS number 81093-37-0, and
Rosuvastatin, with IUPAC name (E,3R,5S)-7-[4-(4-fluorophenyl)-2-[methyl(methylsulfonyl)amino]-6-propan-2-ylpyrimidin-5-yl]-3,5-dihydroxyhept-6-enoic acid and CAS number 287714-41-4.

In a more particular embodiment, the statin for use in the treatment and/or prevention of portal vein thrombosis as described above is simvastatin.

Statins are widely used drugs in the treatment of hyperlipidemia and preventing or reducing cardiovascular diseases. This is advantageous, in that their safe use, even in patients suffering from liver diseases, has been confirmed in several studies. Given that the portal vein is the main blood vessel in the portal venous system and its function is to carry blood from the organs in the abdomen to the liver, it is particularly important that the treatment, in this case statins, is well tolerated by this system and its organs, in particular if there is a dysfunction of any kind.Thus, in a particular embodiment, the statins or their pharmaceutically acceptable salts for use as described above, are those where the prevention and/or treatment is conducted in a patient suffering from a vascular liver disease. Given that PVT is a condition that occurs in a greater percentage in cirrhotic patients, in a more particular embodiment, the statins or their pharmaceutically acceptable salts for use as described above, are those where the vascular liver disease is cirrhosis.

Inventors have identified statins' use in the prevention and/or treatment of PVT by inhibiting one of its main causes, namely PV wall thickening. This is particularly advantageous in that anticoagulants are not a valid therapeutical option for all patients suffering from PVT, since the main driver of this condition is not thrombus formation, but wall thickening. The use of statins not only enables to halt the progression of the disease by inhibiting further EndMT of endothelial cells, but they moreover revert EndMT of endothelial cells, hence showing their capacity of treating and reducing PVT. Thus, in a particular embodiment the statin or the pharmaceutically acceptable salt thereof for use as described above is administered to a patient not responding to anticoagulant treatment. In a more particular embodiment, the statin for use as described above is for use in combination therapy with one or more known anticoagulant agents. In another particular embodiment, the statin or the pharmaceutically acceptable salt thereof for use as described above is administered simultaneously, sequentially or separately with the one or more known anticoagulant agents. In a more particular embodiment, the anticoagulant agent is selected from the following group: low-molecular-weight-heparin, vitamin K antagonist, and direct oral anticoagulants (DOACs).

The use of statins at an early stage in cirrhotic patients prevents them from developing PVT, representing an enormous benefit for these patients. Moreover, due to the particular mechanism of portal vein wall thickening, namely EndMT, statins may be considered the only therapeutical option for patients already suffering from PVT, enabling them to halt the disease and ameliorate their prognosis. In general terms, the use of statins can reduce the threat posed by this condition in cirrhotic patients, given that PVT is a common complication which not only lacks effective treatment, but moreover worsens their prognosis. Some patients suffering from PVT may not even be eligible for liver transplant. Thus, in a particular embodiment, the statin or the pharmaceutically acceptable salt thereof for use as described above is administered to a patient that needs a liver transplant or a patient that has already received a liver transplant.

In another particular embodiment the statin or a pharmaceutically acceptable salt thereof is administered in the form of a pharmaceutical composition which comprises a therapeutically effective amount of statin or a pharmaceutically acceptable salt thereof, together with pharmaceutically acceptable excipients or carriers. In another particular embodiment, the statin or the pharmaceutically acceptable salt thereof, for use as described above is for oral, intravenous and intramuscular administration, as well as targeted delivery to endothelial cells.

In a particular embodiment, the pharmaceutically acceptable excipients or carriers may include diluents (such as lactose monohydrate, talc), extender agents, anticaking agents (such as magnesium stearate), disintegrants (such as croscarmellose sodium), stabilisers, preservatives (such as antioxidants like ascorbic acid, butylhydroxyanisol, etc), buffers, emulsifiers, flavouring agents, colouring agents, sweetening agents, thickeners (such as hydroxypropylcellulose), correctors, film-forming agents, dissolution aids and other additives, generally known per se, such as water, vegetable oil, alcohol (e.g., ethanol or benzyl alcohol, etc.), polyethylene glycol, glyceryl triacetate, gelatine, carbohydrate (e.g. lactose, starch, etc.), magnesium stearate, talc, lanolin, petrolatum and the like.

Statins are metabolized in the liver and this must be taken into consideration in patients suffering from a liver disease, adjusting the dose correspondingly. Thus, in a particular embodiment, the statin or the pharmaceutically acceptable salt thereof, for use as described above, is administered in a dose which is from 1 to 85mg per day, as a single dose. In another particular embodiment, when the statin is simvastatin, the dose administered is from 5 to 45mg per day, as a single dose. In a more particular embodiment, the dose administered is from 10 to 40mg, preferably 20mg per day, as a single dose. In another particular embodiment, the dose administered to a patient is from 20 to 40 mg, wherein the patient is a cirrhotic patient.

In another particular embodiment, when the statin is atorvastatin the dose administered is from 5 to 35mg per day, as a single dose. In a more particular embodiment, the dose administered is from 10 to 30mg, preferably 10 to 20mg per day, as a single dose. In another particular embodiment, when the statin is fluvastatin the dose administered is from 15 to 85mg per day, as a single dose. In a more particular embodiment, the dose administered is from 30 to 80mg, preferably 60 to 80mg per day, as a single dose. In another particular embodiment, when the statin is lovastatin the dose administered is from 15 to 45mg per day, as a single dose. In a more particular embodiment, the dose administered is from 20 to 40mg per day, as a single dose. In another particular embodiment, when the statin is pitavastatin the dose administered is from 1 to 5mg per day, as a single dose. In a more particular embodiment, the dose administered is from 2 to 4mg per day, as a single dose. In another particular embodiment, when the statin is pravastatin the dose administered is from 5 to 45mg per day, as a single dose. In a more particular embodiment, the dose administered is from 10 to 40mg, preferably 20 to 40mg per day, as a single dose. In another particular embodiment, when the statin is rosuvastatin the dose administered is from 5 to 15mg per day, as a single dose. In a more particular embodiment, the dose administered is from 5 to 10mg per day, as a single dose.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### EXAMPLES

Abbreviations: Portal Vein (PV) , extracellular matrix (ECM), portal vein thrombosis (PVT), hepatocelullar carcinoma (HCC), portal vein thrombosis (PVT), polycystic liver disease (PLD), hepatitis C virus (HCV), hepatitis B virus (HBV), hepatitis D virus (HDV), human immunodeficiency virus (HIV), magnetic resonance imaging (MRI), Hematoxylin and eosin (H&E), portal vein endothelial cells (PVECs), control patients with PLD but without signs of portal hypertension (P_CT), cirrhotic patients without PVT (P_CH), cirrhotic patients with PVT (P_PVT), differentially expressed genes (DEGs), transcripts per million (TPM), Drug Gene Interaction Database (DGldb), Short Time-course Expression Miner (STEM), endothelial to mesenchymal transition (EndMT), quantitative real-time polymerase chain reaction (qPCR), fast gene set enrichment analysis (fGSEA), transcription factors (TF), tunica intima (Tl), tunica media (TM).

### Example 1: Portal vein tissue collection and portal vein wall composition analysis

Portal vein and hepatic vein tissues were prospectively collected from explantes livers right after liver hepatectomy of cirrhotic patients with (n=5) or without PVT (n=12), regadless of the underlying etiology or the presence of hepatocelullar carcinoma (HCC). Tissue collection was also performed from explanted livers of PLD patients without signs of portal hypertension (n=3), in order to serve as control group. Exclusion criteria of cirrhotic patients were the presence of PVT due to HCC macrovascular invasion and the patient presentation of active HCV, HBV, HDV or HIV viruses at the time of surgery. PLD patients presenting portal hypertension were also excluded from the study. Tissues were rapidly immersed in EGMTM-2 Endothelial Cell Growth Medium-2 BulletKitTM (EGM-2) (Cultek, #CC-3162) between 10 and 60 minutes after hepatectomy and samples were processed between 1 and 6 h after collection.

PVT was diagnosed by demonstrating presence of endoluminal material compatible with non-tumoral thrombosis in the portal vein and/or its branches at Doppler-US. PVT diagnosis and its extension were always confirmed by computed tomography or magnetic resonance imaging (MRI) and classified according to the latest expert consensus. Patients classified as PVT included those who had a recent history of PVT but with no active thrombi at the time of liver transplantation (recanalization) and those who arrived at surgery with an existing thrombus.

### Example 2: Analysis of portal vein wall composition

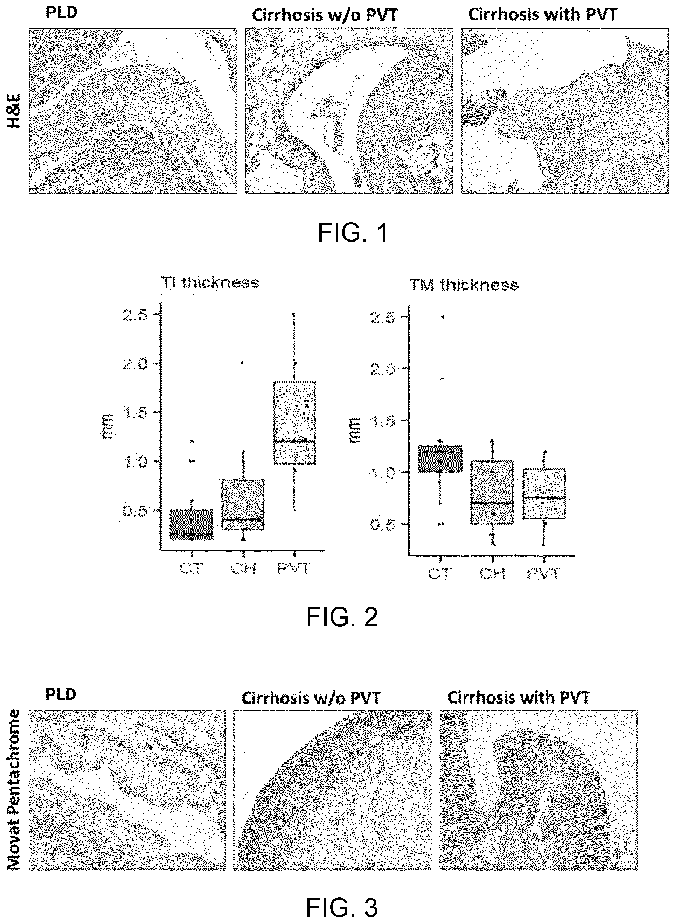

Portal veins and hepatic veins of control PLD patients with no signs of hypertension (CT), cirrhotic patients without PVT (CH) and cirrhotic patients with PVT (PVT) were fixed in either formalin or Paxgene, paraffin embedded and 4.5µm sections obtained. Hematoxylin and eosin (H&E) staining was performed for structural analysis (see FIG. 1). Results showed that during liver damage, tunica intima (TI) suffers from fibrosis and thickening, whilst tunica media (TM) suffers from degeneration (see FIG 2).

Vein wall composition was analyzed by Movat Pentachrome Stain Kit (Modified Russell-Movat, Abcam, #ab245884) to detect elastic fibers, collagen, mucin, fibrin, and muscle (see FIG 3.). Portal vein slides were deparaffinized with xylene and ethanol and antigen retrieval performed with a citrate solution (pH 6). Endogenous peroxidases were inactivated with 3% hydroxide peroxide (Panreac, #1.410.761.211) incubation and slides blocked with antibody diluent with background reducing component at room temperature (Dako, #S3022). Immunostaining of CD68 (1:100, Dako, #M087601-2), CD3 (1:100, Dako, #A045229-2) were performed overnight at 4°C. Primary antibodies were visualized with Dako Real Envision Detection System- HRP (#K4001, #K4003), and slides were counterstained with hematoxylin (Sigma, #GHS232-1L).

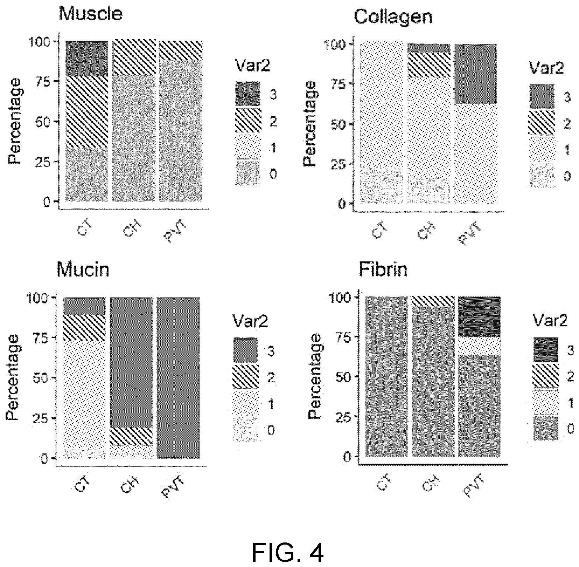

Results showed that differences between the three groups of patients exist and subendothelial layer was identified as the primary driver of this thickening, since it showed an enhanced secretory phenotype, evidenced by an increased mucin and extracellular matrix (ECM) deposition and reorganization. Histological analysis was performed on the inflammatory infiltrates in the portal vein wall to confirm these changes. Collagen, fibrin, mucin, muscle and elastic fibers content were evaluated using a score from 0 to 3 according to the following criteria: 0 = none; 1 = mild; 2 = moderate; 3 = severe (see FIG. 4).

An increase in collagen, mucin and fibrin deposition, together with a decrease in muscle was observed in patients with cirrhosis in comparison to polycystic liver disease (PLD) patients. However, histological changes were even more pronounced in patients with portal vein thrombosis (PVT), suggesting a more advanced remodeling of PV wall occurs with this pathology (see FIG. 4).

### Example 3: Endothelial cells isolation from human portal vein

For portal vein endothelia cell (PVEC) isolation, excess surrounding fat tissue was meticulously removed from the vein using sterile scissors, and any residual blood was washed away with DPBS. Subsequently, the tubular vein was opened to expose the inner part of the vein facing upward. Trypsin EDTA 0,05% (Gibco, #25300054) was then added on the upper surface and incubated for 5 minutes at 37°C. EGM-2 medium was then added abundantly over the tissue and a cell scrapper (TPP-Reactiva, #99002) was used to carefully scrap the surface of the inner wall several times, in order to detach the endothelial cells from the vein wall. The EGM-2 medium with cells in suspension was then centrifuged at 300 g for 5 minutes and pellet resuspended in 2 ml of fresh EGM-2. Cells were then seeded in a well from a 6-well plate pre-coated with human plasma fibronectin at a concentration of 1 µg/cm² (Sigma, #F0895), and fresh media changed every other day until confluence. On average, confluence was reached 10 days after isolation.

In order to isolate PVECs from other cell types that were also scrapped during the initial tissue processing, cells were detached, stained with anti-CD31-FITC (BD Bioscience, #555445) and CD144 -BV786 (BD Bioscience, #565672) antibodies and FACS sorted in a FACS Aria II cell sorter (BD Biosciences) according to manufacturer's instructions. PVECs were selected as CD31⁺ CD144⁺ cells and seeded back to either a 6-well plate, T25 or T75 flasks, depending on the cell amount obtained. Once ECs reached confluency, a third of the cells were collected in RLT buffer (Qiagen) for subsequent RNA isolation (passage 1), a third was used for flow cytometry purity analysis and the final one third was passaged into a new 6-well plate. From this moment onwards, both portal vein endothelial cells (PVECs) were expanded at a split ratio of 1:5 until senescence. At every passage, a fraction of the cells was cryopreserved for future experiments, while the rest was used for flow cytometry purity check, RLT collection and tube assays. Endothelial phenotype of PVECs was routinely monitored by cell morphology, flow cytometry, immunofluorescence and tube formation capacity.

### Example 4: RNA extraction, sequencing and bioinformatic analysis

Passage 1 PVECs P_CT (n = 3), P_CH (n = 12) and P_PVT (n = 5) were collected in RLT + β-mercapto and RNA was isolated with the RNeasy Micro kit (Qiagen) with on-column DNAsel treatment following manufacturer's instructions. RNA purity and quality was analyzed with Bioanalyzer and only samples with a RNA Integrity Number above 8 were used for subsequent steps. Libraries were synthesized with the NEBNext Ultra II Directional RNA Library Prep Kit for Illumina (New England Biolabs) and sequencing was paired end (2x50 bp) on an Illumina NovaSeq 6000 sequencer, obtaining a minimum of 30 million read per sample. Sequence quality was checked with FastQC (version 0.11.9). Reads were pseudoaligned to the human genome (version GRCh38.p13) with the Kallisto aligner. Gene level TPM Counts calculation and differential expression analysis were performed with sleuth R package (version 0.30.1) using only protein-coding genes. Differentially expressed genes (DEGs) were identified using the likelihood ratio test by pvalue < 0.05 and FDR < 0.2. Functional enrichment and drug repurposing analysis were performed with the fgsea R package (version 1.26.0), using the b-statistic as proxy for gene-level stats. Reactome, Kegg and Molecular Signatures (MSigDB, GO Biological Process ontology) databases were used as the source for functional annotation to uncover biological functions related to gene expression changes.To agglomerate gene patterns along disease stages, Short Time-course Expression Miner (STEM) algorithm was used through on-line platform. To identify in an unbiased way the transcription factors predicted to be directly involved on transcriptomic changes we apply functional prediction of differentially expressed genes (DEG) by the use of CheA software from Enrichr platform.

### Example 5: Analysis of PVEC transcriptomic data from different groups of patients

The present inventors performed a comprehensive analysis of the PVEC (portal vein endothelial cells) transcriptomic data from a series of cirrhotic patients without PVT (CH, n=12), cirrhotic patients with PVT (PVT, n=5) and a unique set of explants from patients with polycystic liver disease (PLD) undergoing liver transplantation (PLD, n=3), included as controls since they did not suffer from portal hypertension.

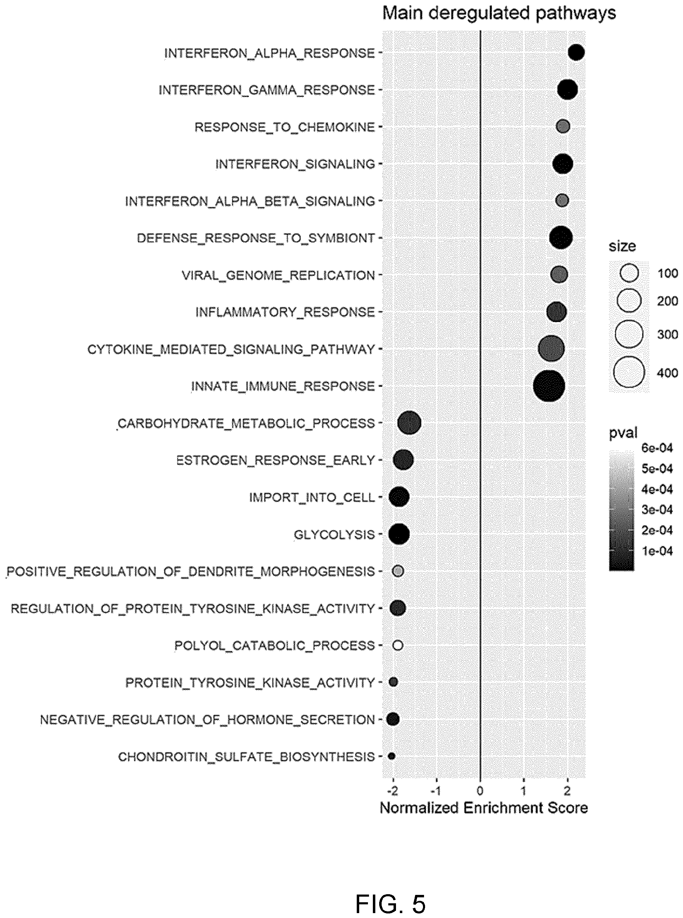

CT-PVECs (from PLD control group) were compared with CH-PVECs (from cirrhotic patients) and the effects of cirrhosis, as well asthe development of portal hypertension on the different PVECs were analysed. Fast gene set enrichment analysis (fGSEA) results showed that endothelial cells from cirrhotic patients undergo significant up-regulation of a variety of immune-related pathways, primarily involving cytokine and chemokine signalling, along with immune cell migration (see FIG. 5).

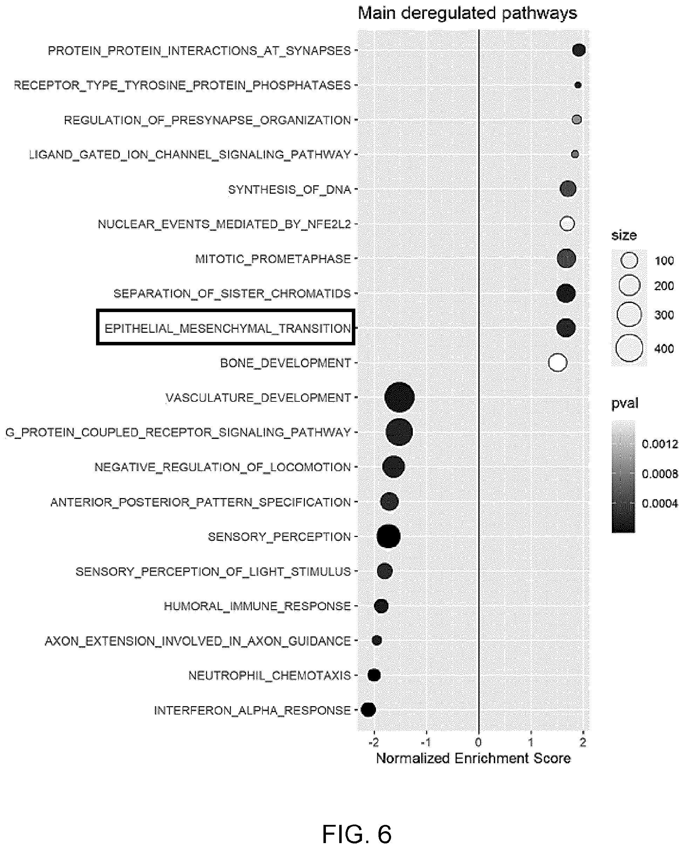

When PVECs from cirrhotic patients without PVT and those with PVT were compared, GSEA revealed up-regulation of genes related to endothelial to mesenchymal transition (EndMT) pathway as one of the most significantly altered processes, together with an increase in ECM production and organization (see FIG. 6). Immune regulation-related pathways were enriched among down-regulated genes as well as GTPases, suggesting CH-PVT-PVECs displayed a more secretory phenotype and increased permeability of the endothelial barrier. Overall, these changes suggest that EndMT could be the driving mechanism behind intimal hyperplasia and TI proliferation.

### Example 6: Hallmark genes expression levels related to occurrence of EndMT in PVECs

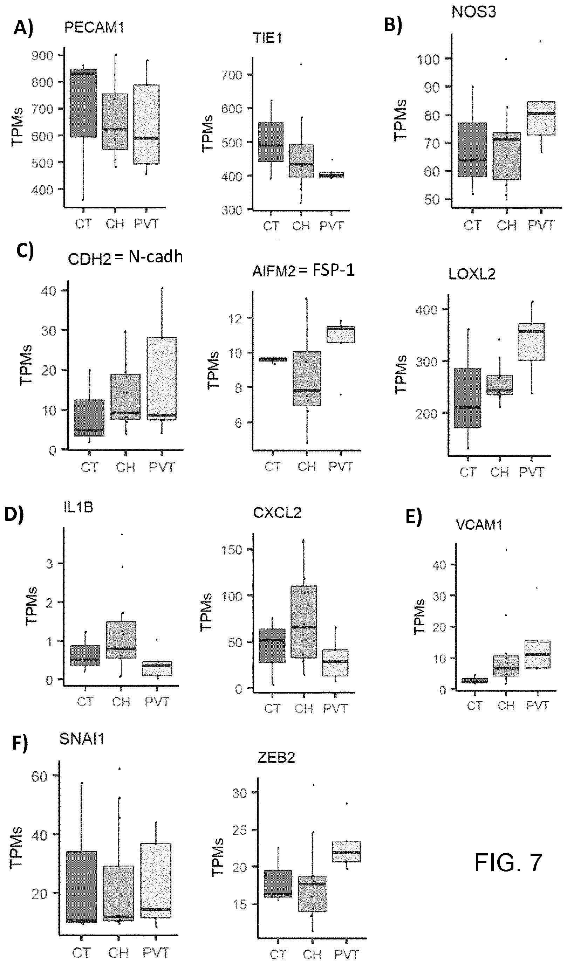

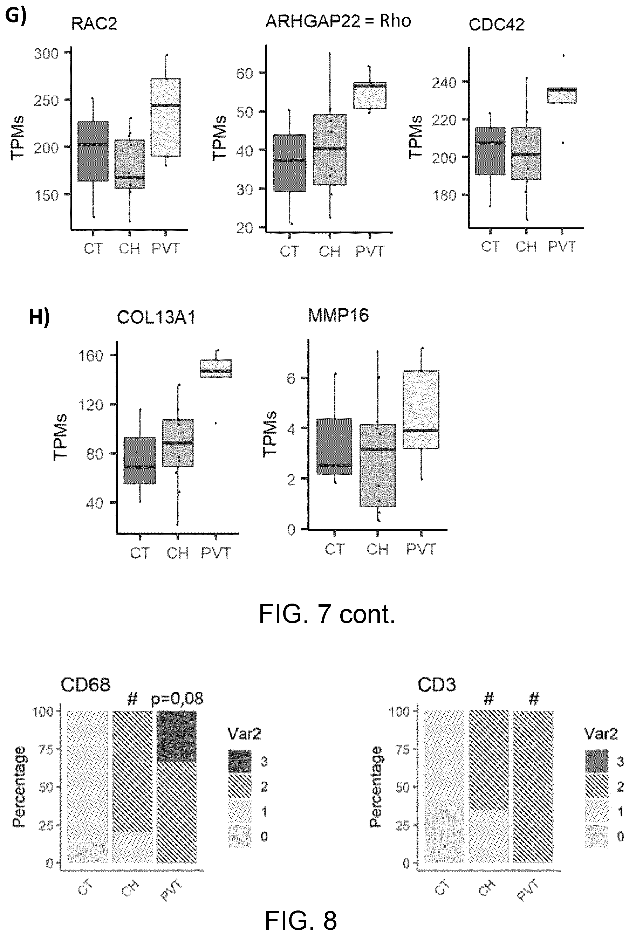

To delve deeper into the EndMT mechanism occurring in PVECs, the present inventors analyzed the expression levels of the EndMT's hallmark genes in the three different groups of patients, CT (PLD control patients without portal hypertension), CH (cirrhotic patients without PVT), and PVT (cirrhotic patients with PVT). Hallmarks of EndMT are known to include: the loss of EC markers and function (PECAM1 and TIE1) (see FIG. 7 A and B), accompanied by the gain in mesenchymal cell markers (N-cadh and FSP-1) and functions (LOXL2) (see FIG. 7 C), deregulation of inflammation (IL 1b and CXCL2) (see FIG. 7 D), increased expression of adhesion molecules (VCAM1) (see FIG. 7 E), increased expression of TF governing EndMT (SNAI1 and ZEB2) (see FIG. 7 F), and finally dramatic cytoskeletal reorganization (triggered by GTPases Rho and Rac) (see FIG. 7 G), ECM remodeling (COL13A1 and MMP16) (see FIG 7 H).

The expression of all these EndMT's hallmark genes were markedly altered in PVT and, most importantly, analysis of these results suggested that these changes tend to progress as the disease advances. Thus, EndMT occurs as a progressive pathological mechanism in portal vein.

The present inventors further analysed which transcription factors (TF) could be driving the coordinated gene expression changes observed during PVECs remodeling. DEGS between CH-PVECS and CH-PVT-PVECs were specifically analysed, and ChEA software was used to compute over-representation of transcription factor targets. The analysis uncovered an increase in predicted upstream activity of mainly SMAD2 and SMAD3 (see Table 1). SMAD transcription factors are the principal downstream effectors of TGF-β signaling, and are able to activate other transcription factors like Snail, which is one of the most important TF in the onset of EndMT.

**Table 1: ChEA results from Enrichr analysis from DEGS from the comparison between CH-PVECS and CH-PVT-PVECs.**

| Index | Term | p-value |
|---|---|---|
| 1 | CTCF 27219007 (Human) | 5.5E-04 |
| 2 | ELF3 26769127 (Human) | 5.8E-0.4 |
| 3 | SMAD 19615063 (Human) | 2.2E-0.3 |
| 4 | SMAD2 18955504 (Human) | 2.8E-0.3 |
| 5 | SMAD3 18955504 (Human) | 2.8E-0.3 |
| 6 | CBP 21632823 (Human) | 3.5E-0.3 |
| 7 | CTCF 27219007 (Human) | 3.9E-0.3 |
| 8 | KLF4 26769127 (Human) | 4.3E-0.3 |
| 9 | P300 27058665 (Human) | 4.5E-0.3 |
| 10 | RUNX 20019798 (Human) | 4.7E-0.3 |

In agreement with the transcriptomic analysis, histological examination of PV revealed a significant increase in immune cell infiltration characterized by the presence of CD68-positive macrophages and CD3-positive leukocytes in the intimal layer of portal veins from cirrhotic patients compared to PLD, which was even more pronounced in the portal vein of cirrhotic patients with PVT (FIG. 8). All these myeloid cells present in the portal vein wall are known to be secreting TGFβ, which could be the main driver of EndMT.

Altogether, these findings suggest that the EndMT occurring in the PV, that ultimately may lead to PVT development, could be the result of increased immune cell infiltration into the vein wall, with the subsequent increased secretion of TGFβ and endothelial activation of the SMAD-Snail pathway.

### Example 7: Evaluation of Simvastatin's effect in vitro on EndMT

PVECs were seeded in 6-well plates in triplicates (for RNA collection) and in 60mm dishes (for protein collection) at a concentration of 6x104 cells/ml and grow for 24 h to obtain 70% confluency. PVECs from CT (n=3), CH (n=7) and CH-PVT (n=6) were then treated *in vitro* with 5 µM Simvastatin sodium salt (Sigma, #567022) for another 24 h and collected in either RLT or RIPA buffer for further analysis by means of quantitative real-time polymerase chain reaction (qPCR) or Western blotting, respectively. Its effect on mesenchymal markers and EndMT promoters, including TFs and all the molecules involved in EndMT initiation or progression previously described, was studied.

Total RNA was isolated by RNeasy Micro and Mini kit (Qiagen) as stated above. First-strand cDNA was synthesized using the High Capacity cDNA Reverse Transcription Kit (Applied, Thermo Fisher, #4368814) according to the manufacturer's instructions. Quantitative real-time PCR (qPCR) was performed using PowerUp^{™} sybr^{®} Green Master Mix (Applied Biosystems, #A25776) with technical duplicates using an AB1 Prism^{®} 7900 HT Cycler (Applied Biosystems). PCR cycle parameters were as follows: 95 °C for 10' followed by 40 cycles at 95 °C for 15" and 60 °C for 1'. Results were obtained with the Sequence Detection System 3.2 software (Applied Biosystems) and further analyzed by the 2-ΔΔCt method. GAPDH was used as a loading control. Results are shown as fold-change.

Proteins were extracted with RIPA buffer complemented with Halt Protease & Phosphatase inhibitors (ThermoFisher) and total protein estimated using Bradford's Reagent (Bio-Rad) following manufacturer's instructions. Cell lysates were prepared in Laemmli samples buffer (Bio-Rad) + β-mercaptoethanol, resolved by SDS polyacrylamide gel electrophoresis (PAGE), transferred onto PVDF membranes and blocked with 5% BSA in TBS + 0.05% Tween20. Extracted proteins were analyzed by western blot. Antibodies used were: pSMAD2 (1/1000, Cell signaling, #3108S), pSMAD3 (1/1000, Rockland, #600-401-919), Smad2 (1/1000, Cell signaling, #5339S), Smad3 (Cell signaling, #9523S) and β-actin (1/2000, Sigma, #A2547) served as house-keeping control. PVDF membranes were incubated overnight with primary antibodies at 4°C, washed and incubated 1 h at room temperature with the corresponding horseradish peroxidase-conjugated secondary antibodies. Membranes were finally washed and developed with Immobilon^{®} Western chemiluminiscent HRP substrate (Millipore). Images were acquired with a LAS-4000 apparatus (Fujifilm, TDI, Alcobendas, Spain) and protein expression was determined by densitometric analysis using the Image Studio Lite software (LI-COR). Results are expressed as relative densitometric values normalized to endogenous control β-actin.

**Table 2: Human primer sequences**

| Gene | Primer name | Primer sequence | SEQ ID NO: |
|---|---|---|---|
| GAPDH | GAPDH Fw | TCGGAGTCAACGGATTTGGT | 1 |
| | GAPDH Rv | TTCCCGTTCTCAGCCTTGAC | 2 |
| Cd31/Pecam1 | CD31 Fw | GAAAGCTGTCCCTGATGCCG | 3 |
| | CD31 Rv | ATGTCCTTCCAGGGATGTGC | 4 |
| NOS3 | NOS3 Fw | CGAGTGAAGGCGACAATCCT | 5 |
| | ENOS Rv | ACAGGACCCGGGGATCAAAA | 6 |
| CDH2 | CDH2 Fw | AGGCTTCTGGTGAAATCGCA | 7 |
| | CDH2 Rv | GCAGTTGCTAAACTTCACATTGAG | 8 |
| AIFM2 | AIFM2 Fw | CATGGTGAGGCAGGTCCAG | 9 |
| | AIFM2 Rv | GGCCACTTGGGAGTGAATGA | 10 |
| VCAM1 | VCAM1 Fw | TGGATAATGTTTGCAGCTTCTCAA | 11 |
| | VCAM1 Rv | AGATGTGGTCCCCTCATTCG | 12 |
| SERPINE1 | SERPINE1 Fw | ACCTCTGAGAACTTCAGGATGC | 13 |
| | SERPINE1 Rv | GGGTGAGAAAACCACGTTGC | 14 |
| RAC2 | RAC2 Fw | GGCCAAGGAGATTGACTCGG | 15 |
| | RAC2 Rv | GCCTCGTCGAACACGGTTT | 16 |
| SNAI1 | SNAI1 Fw | CGAGTGGTTCTTCTGCGCTA | 17 |
| | SNAI1 Rv | CTGCTGGAAGGTAAACTCTGGA | 18 |

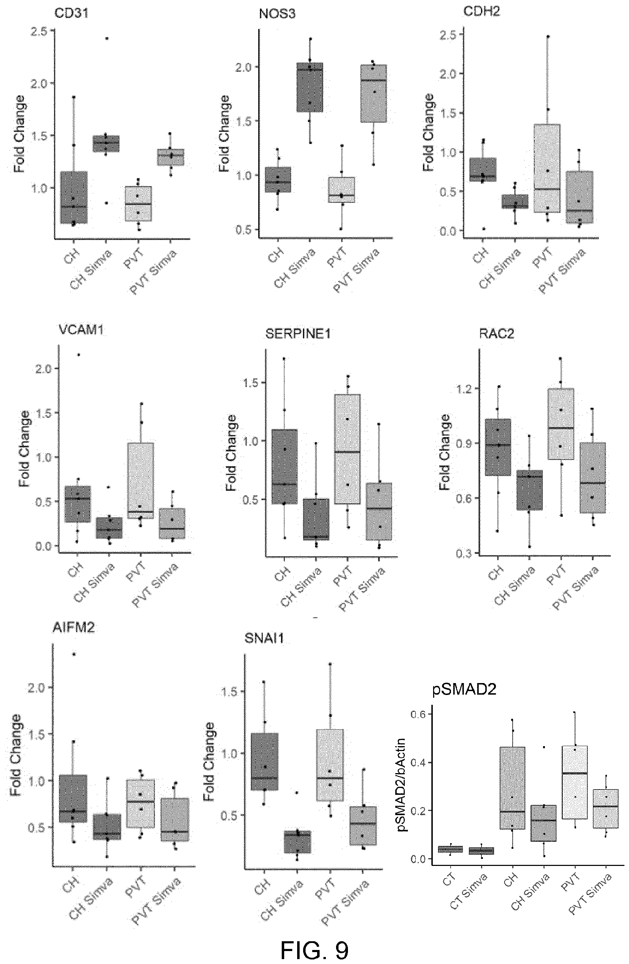

Results demonstrated, for the first time, that Simvastatin can ameliorate the EndMT occurring in our PVEC (see FIG. 9).

qPCR results showed that Simvastatin was capable of increasing endothelial markers expression (Cd31 and NOs3). It also reduced the expression of mesenchymal markers (CDH2 and AIFM2) as well as the expression of the adhesion molecule VCAM1 in PVEC both from cirrhotic patients with or without PVT.Most importantly, it reduced the expression of transcription factor Snail1. Furthermore, Simvastatin exhibited a role in decreasing GTPase (Rac2) and Serpine1 expression. All these markers showing reversion to a more healthy state of PVECS demonstrate Simvastatin can prevent and/or revert the final consequences of EndMT provoking PVT.

### Example 8: Western blotting of PVECs treated with Simvastatin

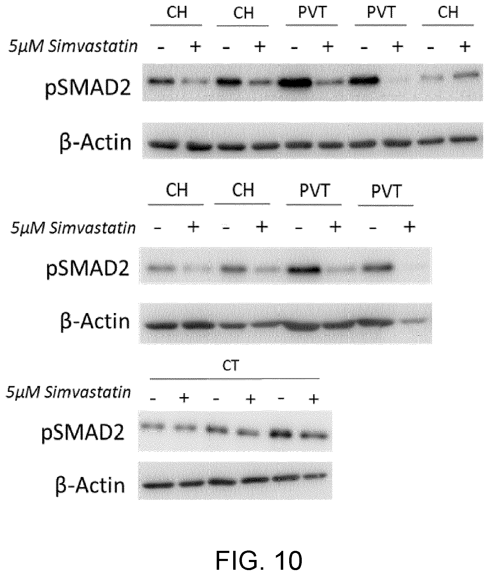

Western blot test was also perfomed on PVECs after been treated *in vitro* with simvastatin. Results (see FIG. 10) showed that simvastatin is capable of reducing phosphor-SMAD2 protein levels. pSMAD2 is a well known downstream effector of TGFb signalling, a key factor in promoting EndMT initiation and progression, that is expressed by all the immune cells demonstrated to be infiltrated in PV wall in cirrhotic patients. Also, western blot analysis confirmed the results obtained in transcriptomic analysis showing that PVECs from ccirhotic patients had more pSMAD2 protein levels than controls.

### Example 9: Exploratory study on cirrhotic patients taking statins

A cohort of cirrohtic patients were prospectively evaluated until the development of portal thrombosis. Some patients were taking statins to treat hypercholesterolemia and hence the difference between these patients and patients not taking statins were analyzed.

It was observed that PVT development was more frequent in patients who did not take statins (see Table 3).

**Table 3: Exploratory study on cirrhotic patients with and without PVT**

| Baseline variables | PVT N = 30 | NO PVT N = 339 |
|---|---|---|
| Age, years - median (IQR) | 58.5 (51.5 - 63.8) | 59 (52 - 67) |
| BMI - median (IQR) | 26.4 (24.8 - 30.5) | 26.9 (24.7 - 29.7) |
| Gender Female - number of patients (%) | 13 (43.3%) | 152 (41.2%) |

| Etiology of liver disease - number of patients (%) | | |
|---|---|---|
| HCV | 8 (26.7%) | 173 (51%) |
| HBV-HBV/HDV | 2 (6.7%) | 16 (4.7%) |
| Alcohol | 8 (26.7%) | 90 (26.5%) |
| Cholestatic + Autoimmune | 2 (6.7%) | 11 (3.2%) |
| NASH | 3 (10%) | 10 (2.9%) |
| Other | 7 (23.3%) | 39 (11.5%) |

| Baseline variables | PVT N = 30 | NO PVT N = 339 |
|---|---|---|
| Child-Pugh score - number (%) | | |
| A | 16 (53.3%) | 250 (73.7%) |
| B | 13 (43.3%) | 67 (19.8%) |
| C | 1 (3.3%) | 22 (6.5%) |
| MELD - median (IQR) | 11 (9.3-13.8) | 9 (7.3 - 11) |
| HCC (number of patients (%) | 0 | 0 |
| Varices - number of patients (%) | 24 (80%) | 198 (58.4%) |
| Ascites (number of patients (%) | 16 (53.3%) | 125 (36.9%) |
| Controlled with diuretics | 7 (25.9%) | 44 (13.4%) |
| Moderate | 8 (29.6%) | 62 (18.8%) |
| Severe | 1 (3.3%) | 19 (5.8%) |
| Encepalopathy (number of patients (%) | 5 (16.7%) | 46 (13.6%) |
| Liver Stifness, KPa - median (IQR) * *available in 34% and 68%* | 24.7 (13.4 - 46) | 23.5 (16.9 - 35.8) |
| HVPG, mmHg - median (IQR) * *available in 59% and 34%* | 17.8 (12.9 - 21) | 15 (12 - 18.5) |
| Splenomegaly - number of patients (%) * *available in 74% of patients* | 15 (71.4%) | 101 (40.1%) |
| Portal flow velocity, cm/sec - mean (± DS) | 15.1 (± 7.6) | 17.9 (± 5.4) |
| Platelets, × 109/L - median (IQR) | 75 (60.5-90) | 106 (75 - 145.5) |
| INR - median (IQR) | 1.3 (1.2 - 1.4) | 1.2 (1.1 - 1.3) |
| Creatinin, mg/dl - median (IQR) | 0.8 (0.7 - 0.9) | 0.82 (0.71 - 0.94) |
| Sodium, mEq/L - median (IQR) | 141 (140 -141.8) | 141 (139 - 142) |
| Bilirubin, mg/dl - median (IQR) | 1.5 (1 - 2.6) | 1.1 (0.8 - 1.6) |
| Albumin, g/L - median (IQR) | 36 (31.3 - 39) | 38 (35 - 42) |
| Statines - number of patients (%) | 1 (3.3 %) | 16 (4.7%) |

### CITATION LIST

### Patent Literature

- WO2018221480A1

### Non Patent Literature

- Nicoar 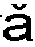 -Farc 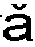 u, O., *et al.,* in "New Insights into the Pathogenesis, Risk Factors, and Treatment of Portal Vein Thrombosis in Patients with Cirrhosis", *Seminars in Thrombosis & Hemostasis,* 2020, Vol. 46, p. 673-681.
- Wan S., et al., in "Systematic review with a meta-analysis: clinical effects of statins on the reduction of portal hypertension and variceal haemorrhage in cirrhotic patients", BMJ Open, 2019, Vol. 9, Issue 7.
- Bhangui, P., et al., in "Current management of portal vein thrombosis in liver transplantation", International Journal of Surgery, Vol. 82, 2020, p.122-127.
- De Franchis, R., et al., in "Baveno VII - Renewing consensus in portal hypertension", Journal of Hepatology, vol. 76, 2022, p. 959-974.
- Anton, A., et al., in "The Pathophysiology of Portal Vein Thrombosis in Cirrhosis: Getting Deeper into Virchow's Triad", Journal of Clinical Medicine, 2022, vol. 11, Article number 800.
- Driever, E. G., et al., in "Nonmalignant portal vein thrombi in patients with cirrhosis consist of intimal fibrosis with or without a fibrin-rich thrombus", Hepatology, 2022, Vol. 75, p. 898-911.

## Claims

1. A statin or a pharmaceutically acceptable salt thereof for use in the prevention and/or treatment of splanchnic vascular dysfuntions in a patient which is a mammal, including a human being, wherein the prevention and/or treatment comprises inhibiting and/or reverting endothelial-to-mesenchymal transition of endothelial cells.

2. The statin or the pharmaceutically acceptable salt thereof for use according to claim 1, wherein the splanchnic vascular dysfuntion is portal vein thrombosis and the endothelial cells are portal vein endothelial cells.

3. The statin or the pharmaceutically acceptable salt thereof for use according to any of the claims 1-2, wherein the inhibition and/or revertion of the endothelial-to-mesenchymal transition of endothelial cells inhibits and/or reverts anatomical changes in the splanchnic vascular system.

4. The statin or the pharmaceutically acceptable salt thereof for use according to any of the claims 1-3, wherein the anatomical change is portal vein wall thickening.

5. The statin or the pharmaceutically acceptable salt thereof for use according to any of the claims 1-4, wherein the prevention and/or treatment further comprises reducing portal vein hypertension.

6. The statin or the pharmaceutically acceptable salt thereof for use according to any of the claims 1-5, wherein the statin is selected from the group consisting of simvastatin, atorvastatin, fluvastatin, lovastatin, pitavastatin, pravastatin and rosuvastatin.

7. The statin or the pharmaceutically acceptable salt thereof for use according to claim 6, wherein the statin is simvastatin.

8. The statin or the pharmaceutically acceptable salt thereof for use according to any of the claims 1- 7, wherein the patient suffers from a splanchnic vascular dysfunctions.

9. The statin or the pharmaceutically acceptable salt thereof for use according to claim 8, wherein the splanchnic vascular dysfunction is cirrhosis.

10. The statin or the pharmaceutically acceptable salt thereof for use according to any of the claims 1- 9, wherein the patient is a patient not responding to anticoagulant treatment.

11. The statin or the pharmaceutically acceptable salt thereof for use according to any of the claims 1-10, wherein the statin or the pharmaceutically acceptable salt thereof is administered simultaneously, sequentially or separately with the one or more known anticoagulant agents.

12. The statin or the pharmaceutically acceptable salt thereof, for use according to any of the claims 1-11, wherein the statin or the pharmaceutically acceptable salt thereof is administered in the form of a pharmaceutical composition which comprises a therapeutically effective amount of the statin or the pharmaceutically acceptable salt thereof, together with pharmaceutically acceptable excipients or carriers.

13. The statin or the pharmaceutically acceptable salt thereof, for use according to any of the claims 1-12, wherein the statin is either orally, intravenously, or intramuscularly administered, or is administered to endothelial cells through targeted delivery system.

14. The statin or the pharmaceutically acceptable salt thereof, for use according to any of the claims 1-13, wherein the statin is administered in a dose which is from 1 to 85mg per day, as a single dose.

15. The statin or the pharmaceutically acceptable salt thereof, for use according to any of the claims 1-14, wherein the statin is simvastatin and it is administered in a dose which is from 20 to 40mg per day, as a single dose.
